# EUROPEAN PATENT SPECIFICATION

(11) **EP 2 691 083 B1**
(45) Date of publication and mention of the grant of the patent: **02.08.2017**
(21) Application number: 12711399.1
(22) Date of filing: 29.03.2012
(51) Int. Cl.: A61K 9/16, A61K 9/20, A61K 31/4985

(54) **PHARMACEUTICAL COMPOSITION OF SITAGLIPTIN**
PHARMAZEUTISCHE ZUSAMMENSETZUNG VON SITAGLIPTIN
COMPOSITION PHARMACEUTIQUE DE SITAGLIPTINE

(30) Priority: 29.03.2011 SI 201100115 P
(43) Date of publication of application: 05.02.2014
(73) Proprietor: KRKA, tovarna zdravil, d.d., Novo mesto, 8501 Novo mesto (SI)
(72) Inventor: PRESKAR, Maja, 8270 Krsko (SI); KROSELJ, Vesna, 8000 Novo mesto (SI); TROST, Sabina, 1330 Kocevje (SI); HVALEC, Lucija, 8000 Novo mesto (SI); KLJAJIC, Alen, 3000 Celje (SI); BUKOVEC, Polona, 8000 Novo mesto (SI)
(74) Representative: Uexküll & Stolberg
(86) International application number: PCT/EP2012/055736
(87) International publication number: WO 2012/131005

(56) References cited:
- WO-A1-2006/033848
- WO-A1-2009/111200
- WO-A1-2011/127106
- WO-A2-2008/000418
- WO-A2-2012/031124
- WO-A2-2012/076973
- US-A1- 2007 172 525
- US-A1- 2008 064 701
- US-A1- 2010 249 140
- BROADHEAD J ET AL: "THE SPRAY DRYING OF PHARMACEUTICALS", DRUG DEVELOPMENT AND INDUSTRIAL PHARMACY, NEW YORK, NY, US, vol. 18, no. 11/12, 1 January 1992 (1992-01-01), pages 1169-1206, XP009030193, ISSN: 0363-9045, DOI: 10.3109/03639049209046327
- PATTERSON J E ET AL: "Preparation of glass solutions of three poorly water soluble drugs by spray drying, melt extrusion and ball milling", INTERNATIONAL JOURNAL OF PHARMACEUTICS, ELSEVIER BV, NL, vol. 336, no. 1, 12 April 2007 (2007-04-12), pages 22-34, XP022026284, ISSN: 0378-5173, DOI: 10.1016/J.IJPHARM.2006.11.030

## Description

### FIELD OF THE INVENTION

The present invention relates to stable pharmaceutical compositions comprising amorphous sitagliptin and to processes for their preparation.

### BACKGROUND OF THE INVENTION

Sitagliptin, a compound of formula I, is a dipeptidyl-peptidase-4 (DPP-4) inhibitor and is used for treatment of diabetes mellitus type 2, also known as non-insulin dependent diabetes mellitus.

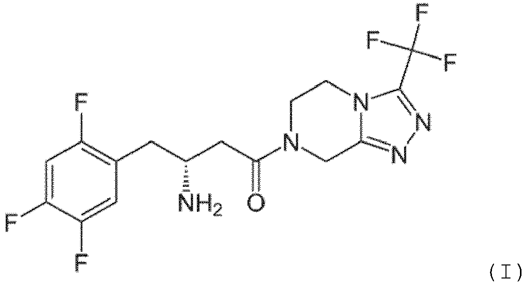

Sitagliptin is currently marketed in the form of film coated tablets under the trade name Januvia^{™} 25, 50 and 100 mg in United States and European Union. The currently marketed formulation contains sitagliptin in the form of sitagliptin phosphate monohydrate, microcrystalline cellulose, anhydrous calcium hydrogen phosphate, croscarmellose sodium, magnesium stearate and sodium stearyl fumarate. The film coating contains polyvinyl alcohol, macrogol 3350, talc, titanium dioxide and colorants.

Sitagliptin base and its pharmaceutically acceptable acid addition salts have been described in WO03004498. In particular, Example 7 WO03004498 discloses the preparation of sitagliptin base and its hydrochloride salt.

WO2005003135 describes dihydrogenphosphate salt of the dipeptidyl peptidase-IV inhibitor sitagliptin and crystalline hydrates thereof, in particular a crystalline monohydrate.

New salts and polymorphs of sitagliptin or its salts are described in WO2005020920, WO2005030127, WO2006033848, WO2005072530, WO2007035198, KR20070111099, WO2009084024, WO2009070314, WO2009085990, WO2009120746, US2009247532, WO2010000469, WO2010012781, WO2010032264, WO2010092090, US2010249140, WO2010122578, and WO2010131035.

The bioavailability of a therapeutically active compound is generally affected by the solubility/dissolution rate of the compound, and the partition coefficient/permeability of the compound through a subject's gastrointestinal membrane. The major cause of poor bioavailability of a therapeutically active compound is the poor solubility/dissolution rate of said compound. Poor bioavailability is also often accompanied with undesirably high rates of patient variability and unpredictable dose/therapy effects due to erratic absorption of the therapeutically active compound by the patient.

The crystalline state of the active ingredient in a solid state pharmaceutical preparation may play a significant role in the behavior of the drug, once taken orally, and may influence its therapeutic effect. The crystalline state may modify the dissolution and thus influence absorption and the therapeutic effect of the drug.

According to the literature data, amorphous forms are generally more hygroscopic and less stable than crystalline forms and crystallize into a crystalline form or a mixture of crystalline forms (Brittain HG. Polymorphism in Pharmaceutical Solids. 1st ed. New York: Marcel Dekker, 1999). For example, amorphous forms of indomethacin may be stabilized by using a solid dispersion of indomethacine in PVPP (Chem. Pharm Bull. 1983; 31 (7): 2510-2512).

The journal reference Phar. Devel. Techn, 11:521-528,2006 by S. Fitzpatrick discloses pharmaceutical compositions comprising crystalline sitagliptin. According to this article, amorphous sitagliptin has a poorer stability profile than the anhydrous crystalline form and formation of the amorphous form should therefore be prevented during preparation of the pharmaceutical composition.

WO2006033848 teaches pharmaceutical compositions comprising amorphous sitagliptin phosphate prepared by a direct compression process. However, despite great efforts the present inventors have not been able to prepare stable pharmaceutical composition according to WO2006033848 because the amorphous sitagliptin phosphate in the tablets crystallized.

Therefore, it is the object of the present invention to provide a pharmaceutical composition comprising sitagliptin in a stable and readily bioavailable form.

### DETAILED DESCRIPTION OF THE INVENTION

The present invention discloses stable pharmaceutical compositions comprising amorphous sitagliptin and how to prepare them. The term "sitagliptin" according to present invention comprises sitagliptin hydrochloride, except when explicitly noted otherwise. It has been tried to prepare tablets with amorphous sitagliptin according to the method disclosed in WO2006033848 by a direct compression method, however without success. After considerable experimentation, it has surprisingly been found that the stability of amorphous sitagliptin is mostly influenced by the presence of a crystallization inhibitor as well as the process for its preparation. In particular, it was found that stable amorphous sitagliptin can be prepared from a solution comprising sitagliptin and a crystallization inhibitor.

Hence, the present invention relates to the pharmaceutical composition according to claim 1 comprising amorphous sitagliptin, wherein amorphous sitagliptin has been prepared from a solution comprising sitagliptin and a crystallization inhibitor, and wherein the composition further comprises a pharmaceutically acceptable excipient which is microcrystalline cellulose. According to one aspect, the present invention relates to the pharmaceutical composition according to claim 1 comprising amorphous sitagliptin, a crystallization inhibitor and a pharmaceutically acceptable excipient which is microcrystalline cellulose. Furthermore, the present invention relates to the process according to claim 7 for preparing the pharmaceutical composition according to the invention. Said process for preparing the pharmaceutical composition comprises
a) providing a solution comprising sitagliptin and a crystallization inhibitor in a solvent; and
b) converting the solution provided in step a) into a particulate form.

A process for the preparation of the pharmaceutical composition according to the present invention can comprise the following steps:
a) preparing a solution of sitagliptin and a crystallization inhibitor in a solvent; and
b) loading the obtained solution on the pharmaceutically acceptable excipient. Alternatively, the process for the preparation of the pharmaceutical composition according to the present invention can comprise the following steps:
   a) preparing a solution of sitagliptin and a crystallization inhibitor in a solvent;
   b) spray drying the obtained solution to form a solid; and
   c) mixing the obtained solid with the pharmaceutically acceptable excipient. As used herein, the term "sitagliptin" refers to sitagliptin hydrochloride. The sitagliptin hydrochloride salt can be prepared either in situ by adding an HCl solution to a dispersion of sitagliptin base, or vice versa, by dissolving sitagliptin base in an HCl solution. The molar ratio of sitagliptin base to acid in a solution can be in the range of 1:0.5 to 1:5, preferably in the range of 1:0.5 to 1:3. The pH value of the sitagliptin hydrochloride salt solution can be between 2 and 6, preferably 3.0 to 5.5.

The crystallization inhibitor is polyvinylpyrrolidone. It can be present in a range of 0.1 to 15 weight%, preferably 1 to 10 weight% based on the weight of the solid pharmaceutical composition. Hence, the pharmaceutical composition according to the invention preferably comprises 0.1 to 15 wt.-%, more preferably 1.0 to 10 wt.-% and most preferably 2.0 to 8 wt.-% of the crystallization inhibitor, based on the total weight of the pharmaceutical composition.

Moreover, it is preferred that the pharmaceutical composition according to the invention is characterized by a weight ratio of sitagliptin to crystallization inhibitor ranging from 1:0.01 to 1:1, preferably 1:0.05 to 1:0.8 and more preferably 1:0.1 to 1:0.5.

Furthermore, it is preferred that the amount of sitagliptin in the solution comprising sitagliptin and crystallization inhibitor that is used for preparing the pharmaceutical composition according to the invention, ranges from about 10 to 50 vol.-%, more preferably 15 to 45 vol.-%, in particular if the solution is loaded on the pharmaceutical excipient by a spray process. Likewise, the amount of crystallization inhibitor in said solution preferably ranges from about 1 to 6 vol.-%, more preferably 2 to 5 vol.-%, in particular if the solution is loaded on the pharmaceutical excipient by a spray process.

The pharmaceutically acceptable solvent used in the preparation of the sitagliptin solution comprising sitagliptin and crystallization inhibitor can be selected from the group consisting of water, alcohol and/or mixtures thereof. The alcohol can be selected from methanol, ethanol, isopropanol and/or mixtures thereof. Preferably, the solvent is water. When the active ingredient is dissolved during the technological process, the particle size of sitagliptin and crystallization inhibitor does not influence the characteristics of the final formulation. However, for process reasons, the preferred number-average particle size of sitagliptin used for providing the solution comprising sitagliptin and crystallization inhibitor is less than 300 µm, preferably less than 200 µm, most preferably less than 150 µm, and in particular ranges from 1 to 300 µm, such as 2 to 200 µm or 10 to 150 µm.

The term "number-average particle size" as used herein refers to the longest dimension of the particles measured using optical or scanning microscope (image analysis). Thus, the term "number-average particle size" refers to the arithmetic mean (count mean diameter, i.e. number-based particle size) which is the summation of all longest dimensions of a given population divided by the number of particles.

Micronized sitagliptin base can be obtained for instance by single or multistage micronization/milling in the dry state using dry mills such as cutting mills, pin/cage mills, hammer mills, jet mills, fluidized bed jet mills, ball mills and roller mills.

Generally, particle size distribution of e.g. sitagliptin can also be measured by laser diffraction method using e.g. a Mastersizer 2000 Hydro S with Isopar L as dispersion medium giving the volume mean diameter, d(90) or d(100) in µm. As used herein, the term "volume mean diameter" refers to the D[4,3] value obtained by a laser diffraction method using a Mastersizer 2000 Hydro S with Isopar L as dispersion medium. For process reasons, the preferred volume mean diameter of sitagliptin used for providing the solution comprising sitagliptin and crystallization inhibitor is less than 300 µm, preferably less than 200 µm, most preferably less than 150 µm, and in particular ranges from 1 to 300 µm, such as 2 to 200 µm or 10 to 150 µm.

The amount of the active ingredient sitagliptin present in the solid pharmaceutical composition according to the present invention can be 1-300 mg, preferably 5-200 mg and most preferably 10-100 mg of sitagliptin per final dosage form. It is preferred that the pharmaceutical composition according to the invention comprises 1 to 50 wt.-%, preferably 10 to 35 wt.-%, more preferably 20 to 30 wt.-% of sitagliptin, based on the total weight of the composition.

The solid pharmaceutical composition according to the present invention can be present in the form of tablets, orodispersible tablets, pills, powders, lozenges, sachets, soft and hard gelatine capsules (filled with powders, granules or pellets), suppositories etc. Preferably, the dosage form is suitable for oral application, most preferably the dosage form is a tablet like a homogeneous single layer tablet. The pharmaceutical composition can comprise one or more further pharmaceutically acceptable excipients. Suitable pharmaceutically acceptable excipients can be selected from (but are not limited to) the group consisting of surfactants, diluents, binders, disintegrants, lubricants, glidants, antioxidants and optionally flavoring and/or sweetening agents. Optionally, the solid pharmaceutical composition can be further coated.

Examples of suitable surfactants include anionic surfactants such as sodium lauryl sulfate and docusate sodium, cationic surfactants such as cetrimide, ampholytic surfactants such as N-dodecyl-N,N-dimethylbetaine, non-ionic surfactants such as sorbitan fatty acid esters (e.g. Spans^{®}), polysorbates (e.g. Tweens^{®}), polyoxyethylene alkyl ethers, poloxamers, medium chain triglycerides, polyoxylglycerides, polyoxyethylene castor oil derivates (e.g. Cremophor^{®}) and mixtures thereof.

The diluent can be selected from the group consisting of but not limited to microcrystalline cellulose, powdered cellulose, composite materials combining crystalline cellulose with lactose, such as combinations comprising α-lactose and powdered cellulose (Cellactose, Tablettose), guar gum (Avicel CE15), silicified cellulose (Prosolv), corn starch, maize starch, starch derivatives such as pregelatinized starch, calcium hydrogen phosphate in anhydrous and hydrated form, various types of sugars such as lactose (anhydrous and monohydrate), compressible sugar, fructose, dextrates, sugar alcohols such as mannitol, sorbitol, maltitol, xylitol, lactitol, and/or other sugars such as saccharose, raffinose, trehalose, fructose or mixtures thereof, calcium carbonate, calcium lactate and/or mixtures thereof. The diluent can be present in an amount of 10 to 99 weight%, preferably 25 to 90 weight% calculated on the weight of the solid composition. Preferably, the pharmaceutical composition comprises one or more diluents selected from the group consisting of microcrystalline cellulose, lactose monohydrate, mannitol and mixtures thereof. Furthermore, it is preferred that the pharmaceutical composition comprises 10 to 99 wt.-%, more preferably 25 to 90 wt.-%, most preferably 40 to 70 wt.-% such as 55 to 65 wt.-% of a diluent, based on the total weight of the pharmaceutical composition.

The binder can be selected from the group consisting of but not limited to polyvinylpyrrolidone, microcrystalline cellulose, cellulose ether, hydroxyethyl cellulose, hydroxypropyl cellulose, hydroxypropyl methylcellulose of different grades (i.e. viscosity), corn starch, maize starch, pregelatinised starch, polymethacrylate, or mixtures thereof. The binder can be present in a range of 0.5 to 25 weight%, preferably 1 to 20 weight% calculated on the weight of the solid composition.

The disintegrant can be selected from the group consisting of but not limited to crospovidone, starch, starch derivatives such as pregelatinised starch and sodium starch glycollate, microcrystalline cellulose, carboxymethylcellulose sodium (CMC-Na, croscarmellose sodium) or calcium (CMC-Ca), crosslinked CMC-Na, polacrilin potassium, low-substituted hydroxypropyl cellulose and/or mixtures thereof and can be present in an amount of 1 to 50 weight%, preferably 2 to 45 weight% based on the weight of the solid pharmaceutical composition. Preferably, the pharmaceutical composition comprises croscarmellose sodium as disintegrant.

The lubricant can be selected from the group consisting of but not limited to stearic acid, magnesium stearate, magnesium palmitate, magnesium oleate, magnesium lauryl sulfate, hydrogenated vegetable oil, hydrogenated castor oil, talc, sodium stearyl fumarate, glyceryl behanate, macrogols and/or mixtures thereof and can be present in a range of 0.1 to 10 weight%, preferably 0.5 to 5 weight% based on the weight of the solid pharmaceutical composition. Preferably, the pharmaceutical composition comprises a lubricant selected from the group consisting of magnesium stearate and sodium stearyl fumarate.

The glidant can be selected from the group consisting of but not limited to colloidal silicon dioxide, talc, stearic acid, palmitic acid, polyethylene glycol, carnauba wax and/or mixtures thereof.

The sweetener can be selected from the group consisting of but not limited to acesulfame K, sucralose, alitame, aspartame, saccharin sodium, dipotassium glycyrrhizinate, stevia and thaumatin and the like.

The flavouring agent can be selected from the group consisting of but not limited to natural or synthetic materials, such as orange, spearmint, strawberry and cream flavour and the like. Preferably, the excipients are selected from the group consisting of lactose, mannitol, carboxymethylcellulose sodium, microcrystalline cellulose and/or mixtures thereof.

The composition may further comprise one or more antioxidants selected from the group consisting of but not limited to alkyl gallates (e.g. dodecyl-, ethyl-, octyl-, propyl-gallate), butylated hydroxyanisole, butylated hydroxytoluene, tocopherols (e.g. alpha tocopherol), ascorbic acid palmitate, ascorbic acid, sodium ascorbate, potassium and sodium salts of sulphurous acid (e.g. bisulphites, metabisulphites, sulphites), flavonoides (rutin, quercetin, caffeic acid). Preferably, the pharmaceutical composition comprises butylated hydroxytoluene as an antioxidant. The composition according to the present invention is the composition of claim 1, wherein sitagliptin is sitagliptin hydrochloride, the crystallization inhibitor is polyvinylpyrrolidone and the pharmaceutically acceptable excipient is microcrystalline cellulose.

The most preferred composition according to the present invention is a composition, wherein sitagliptin is sitagliptin hydrochloride, the crystallization inhibitor is polyvinylpyrrolidone, the pharmaceutically acceptable excipient is microcrystalline cellulose and the composition is a homogeneous single layer tablet.

According to a particularly preferred embodiment, the pharmaceutical composition according to the invention comprises, based on the total weight of the composition,
(A) 10 to 35 wt.-% of sitagliptin hydrochloride,
(B) 1 to 10 wt.-%, preferably 2 to 8.0 wt.-%, of a crystallization inhibitor, which is polyvinylpyrrolidone,
(C) 20 to 50 wt.-%, preferably 30 to 35 wt.-%, of microcrystalline cellulose,
(D) 10 to 40 wt.-%, preferably 20 to 30 wt.-%, of a diluent selected from the group consisting of lactose, mannitol, calcium hydrogen phosphate and maize starch, preferably lactose and/or mannitol,
(E) 1 to 15 wt.-%, preferably 3.5 to 8 wt.-% of croscarmellose sodium,
(F) 0.5 to 5 wt.-% of a lubricant, preferably magnesium stearate and/or sodium stearyl fumarate,
(G) 0 to 0.05 wt.-%, preferably 0.01 to 0.04 wt.-% of an antioxidant, preferably butylated hydroxytoluene, and
(H) optionally further excipients.

The composition is optionally film coated. Film coating can be used to provide improved surface smoothness and color, increased chemical and physical stability of the active agent due to reduced permeability for gases such as oxygen and/or water vapor, less disintegration of the solid composition in acidic medium resulting in decreased gastrointestinal side effects, and easier swallowing of tablet. The film coating comprises coating materials generally known in the art. Suitable coating materials include polymers such as hydroxypropyl cellulose, hydroxypropyl methylcellulose, ethylcellulose, povidone and copovidone, graft copolymers of polyethyleneglycol and polyvinyl alcohol (Kollicoat IR), shellac, polymers of methacrylic acid or methacrylic acid esters, methacrylic acid-methyl acrylate copolymers, polyvinyl alcohol, plasticizers such as propylene glycol, polyethylene glycol, glycerol triacetate, triethyl citrate, antitacking agents such as talc, glycerol monostearate, magnesium stearate and colorants or pigments such as titanium dioxide or iron oxide.

All compositions according to the invention can be packaged into various kinds of containers. Thus, the present disclosure also relates to a packaged pharmaceutical composition comprising the solid pharmaceutical composition described above packaged in a container made of glass or polypropylene with or without desiccant or in a blister made of single or multiple polymer and/or aluminum layers. According to a particular embodiment, the packaged composition is packaged with an atmosphere having a reduced oxygen concentration such as below 15 vol. %, particularly below 10 vol. %, more preferably below 5 vol. % oxygen. According to a particularly preferred embodiment, the composition is packaged with a nitrogen atmosphere.

The composition according to a particular embodiment can be packed into a primary packaging having decreased permeability for water such as high density polyethylene (HDPE) containers with closure, such as polypropylene (PP) closure, and with or without a desiccant; aluminum foil blisters; polychlorotrifluoroethylene (Aclar®) blisters or any other suitable water vapor low permeable packaging such as blisters made of multilayer polymeric foil where different polymeric materials are combined.

The present disclosure also relates to a pharmaceutical product comprising the pharmaceutical composition according to the invention and an adsorbent material. Suitably, the adsorbent material can be a part of the package of the pharmaceutical composition or can be contained in the package of the pharmaceutical composition. Hence, the present disclosure also relates to a stabilized pharmaceutical product comprising a package and packaging method that utilizes an adsorbent material, such as a molecular sieve, that adsorbs moisture in the inner local environment of an impermeable package, so as to prevent hydrolysis and subsequent oxidation products. The adsorbent material can be located in a variety of places. For example, the adsorbent material can be a part of blister foil or can be placed within a porous sachet that, in turn, is located within the sealed package. It is appreciated that numerous adsorbent materials have applications in a stable pharmaceutical product or a method of the present invention, including a molecular sieve, an activated clay, charcoal, an activated alumina, silica, a zeolite, a bauxite, or any mixture of these materials, to name but a few. An effective amount of the adsorbent material used in a stable pharmaceutical product or in a method of the present invention is an amount sufficient to reduce or eliminate the formation of degradation products. One of ordinary skill can readily determine this amount for a particular embodiment of the present disclosure using routine laboratory techniques.

Moreover, a sealed package of a stable pharmaceutical product or a method of the present invention can be produced from a variety of materials, e.g. metal, glass, plastic, etc. Similarly, the shape of a sealed package can vary. Examples of such shapes include, but certainly are not limited to bottle, a bag, a drum box, and an irregularly shaped container. The sealing of a package of a stable pharmaceutical product or a method of the present invention can be accomplished in a variety of ways. More specifically, heat-sealing, gluing, welding, brazing, mechanical closures, mechanical clamps, or compression can hermetically seal a sealed package of a stable pharmaceutical product of the present invention.

During the packaging and/or storing of the pharmaceutical composition according to the invention controlled conditions with low humidity can be used. Additionally inert gases such as nitrogen, argon or helium; or vacuum can be used during the manufacturing of the solid oral dosage forms, the manufacturing of starter, isolated and final pellets including granules and active particles as well as during their packaging to assure inert atmosphere around the solid oral dosage form, such as tablet, pellet or capsule, in the sealed primary packaging. "Inert atmosphere", as used herein, refers to a concentration of oxygen in the atmosphere around the solid dosage form packed in the primary packaging of less than 10 vol%, preferably less than 5 vol% and most preferably less than 2 vol%. The concentration of oxygen in the atmosphere surrounding the pellets, sachets or capsules can be determined by gas chromatography.

The above described methods for preparing the pharmaceutical composition according to the invention include the step of providing a solution comprising the sitagliptin and the at least one crystallization inhibitor in a solvent. According to one embodiment, said solution can further comprise an antioxidant, preferably an antioxidant as described above and/or a surfactant , preferably a surfactant selected from the group consisting of anionic surfactants such as sodium lauryl sulphate and docusate sodium, cationic surfactants such as cetrimide, ampholytic surfactants such as N-dodecyl-N,N-dimethylbetaine, non-ionic surfactants such as sorbitan fatty acid esters (e.g. Spans^{®}), polysorbates (e.g. Tweens^{®}), polyoxyethylene alkyl ethers, poloxamers, medium chain triglycerides, polyoxylglycerides, polyoxyethylene castor oil derivates (e.g. Cremophor^{®}) and mixtures thereof. Preferably, the surfactant is sodium lauryl sulphate. According to another embodiment, the solution substantially consists of the sitagliptin, the crystallization inhibitor and the solvent.

In step b), the solution provided in step a) is converted into a particulate form. In one embodiment, the solution is loaded on one or more pharmaceutically acceptable excipients. In particular, the solution is loaded on one or more pharmaceutically acceptable excipients described above, such as one or more excipients selected from the group consisting of diluents, binders and disintegrants, like the preferred diluents, binders and disintegrants described above. Moreover, such loading can be performed by spraying the solution onto the one or more pharmaceutically acceptable excipients to obtain the excipients coated with a coating comprising amorphous sitagliptin and the crystallization inhibitor. Preferably, the rotor speed applied in the spray process ranges from about 0.5 to 30 rpm, more preferably 1 to 10 rpm. Moreover, it is preferred that the atomization pressure applied in the spray process ranges from about 0.25 to 5 bar, more preferably 0.8 to 2 bar. It is also preferred that the temperature applied ranges from about 25 to 50°C, more preferably 30 to 45°C. The obtained coated particles can then be dried and sieved. Then, the coated particles can be mixed with further excipients such as another part of the disintegrant and/or diluent and/or one or more lubricants. The obtained mixture can then be compressed into tablets.

In another embodiment, the solution provided in step a) can be converted in a particulate form by spray drying. In particular, the solution is sprayed into a stream of hot air to evaporate the solvent and obtain dried particles comprising amorphous sitagliptin and crystallization inhibitor.

The pharmaceutical composition according to the invention as well as the method according to the present invention are advantageous over the known prior art in that, unexpectedly and surprisingly, a highly stable amorphous form of sitagliptin with a low content of impurities in the pharmaceutical composition is obtained.

The invention is illustrated by reference to the following examples. However, the examples are not intended to limit the scope of the claims anyway.

Comparative examples which are based on the process according to WO2006033848 are not part of the present invention.

### EXAMPLES

**Comparative Examples 1-4:**

| Example | 1 | 2 | 3 | 4 |
|---|---|---|---|---|
| Sitagliptin base, milled | 100.0 | 100.0 | 100.0 | 100.0 |
| Sodium lauryl sulphate | 10.0 | 10.0 | 10.0 | 10.0 |
| Lactose spray dried | 150.0 | 150.0 | | |
| Mannitol | | | 150.0 | 150.0 |
| Calcium hydrogen phosphate | 105.0 | | 105.0 | |
| Croscarmellose sodium | 10.0 | 10.0 | 10.0 | 10.0 |
| Microcrystalline cellulose | | 105.0 | | 105.0 |
| Crospovidone | 15.0 | 15.0 | 15.0 | 15.0 |
| Magnesium stearate | 10.0 | 10.0 | 10.0 | 10.0 |
| Tablet coating | | | | |
| Opadry pink | 18.0 | 18.0 | 18.0 | 18.0 |
| Total [mg/tablet] | 418 | 418 | 418 | 418 |

Sitagliptin base was micronized using an air-jet mill with an inlet air pressure of about 14 bar and a milling pressure of about 15 bars. Number-average particle size of the material was below 20 microns.

Micronized sitagliptin base was dry mixed with the other excipients. The resulting mixture was directly compressed to form tablets, which are film- coated using Opadry Pink.

**Comparative Examples 5-8:**

| Example | 5 | 6 | 7 | 8 |
|---|---|---|---|---|
| Sitagliptin base, co-milled with Mannitol (1:1) | 200.0 | 200.0 | 200.0 | 200.0 |
| Sodium lauryl sulphate | 10.0 | 10.0 | 10.0 | 10.0 |
| Lactose spray dried | 150.0 | 150.0 | | |
| Mannitol | | | 150.0 | 150.0 |
| Calcium hydrogen phosphate | 105.0 | | 105.0 | |
| Croscarmellose sodium | 10.0 | 10.0 | 10.0 | 10.0 |
| Microcrystalline cellulose | | 105.0 | | 105.0 |
| Crospovidone | 15.0 | 15.0 | 15.0 | 15.0 |
| Magnesium stearate | 10.0 | 10.0 | 10.0 | 10.0 |
| Tablet coating | | | | |
| Opadry pink | 18.0 | 18.0 | 18.0 | 18.0 |
| Total [mg/tablet] | 518 | 518 | 518 | 518 |

Sitagliptin base and mannitol were co-milled using an air-jet mill with an inlet air pressure of about 14 bar and a milling pressure of about 15 bars. The volume mean diameter of the obtained co-milled material was less than 20 microns (as measured with Malvern Mastersizer 2000).

Co-milled sitagliptin base and mannitol were dry mixed with the other excipients. The resulting mixture was directly compressed to form tablets, which are film coated using Opadry Pink.

**Comparative Examples 1-4:**

| Example | 1 | 2 | 3 | 4 |
|---|---|---|---|---|
| Sitagliptin base, milled | 100.0 | 100.0 | 100.0 | 100.0 |
| Hydroxypropyl methylcellulose 5cP | 30.0 | 30.0 | 30.0 | 30.0 |
| Sodium lauryl sulphate | 5.00 | 5.00 | 5.00 | 5.00 |
| Lactose monohydrate | 100.0 | 100.0 | | |
| Mannitol | | | 100.0 | 100.0 |
| Croscarmellose sodium | 6.50 | 12.50 | 6.50 | 12.50 |
| Microcrystalline cellulose | 138.5 | 138.5 | 136.5 | 136.5 |
| Croscarmellose sodium | 10.0 | 20.0 | 10.0 | 10.0 |
| Magnesium stearate | 10.0 | 10.0 | 10.0 | 10.0 |
| Tablet coating | | | | |
| Opadry pink | 18.0 | 18.0 | 18.0 | 18.0 |
| Total [mg/tablet] | 418 | 434 | 418 | 434 |

Hydroxypropyl methylcellulose and sodium lauryl sulphate were dissolved in water. Sitagliptin base was micronized in accordance with Comparative Examples 1 to 4 and the micronized sitagliptin was dissolved in said aqueous solution. The solution thus prepared was sprayed onto the original mixture of lactose monohydrate or mannitol, a part of the croscarmellose sodium and microcrystalline cellulose. After drying and sieving the other part of croscarmellose sodium and magnesium stearate were added and mixed. The resulting mixture was directly compressed to form tablets, which are film-coated using Opadry Pink.

**Examples 5-12:**

| Example | 5 | 6 | 7 | 8 | 9 | 10 | 11 | 12 |
|---|---|---|---|---|---|---|---|---|
| Sitagliptin base | 100.0 | 100.0 | 100.0 | 100.0 | 100.0 | 100.0 | 100.0 | 100.0 |
| 0.5 M HCl | 2.0 | 2.0 | 2.0 | 2.0 | 2.0 | 2.0 | 2.0 | 2.0 |
| Povidone K25 | 12.0 | 12.0 | 12.0 | 12.0 | 12.0 | 12.0 | 12.0 | 12.0 |
| Calcium hydrogen phosphate | | | 123.0 | 123.0 | | | | |
| Lactose monohydrate | 123.0 | 123.0 | | | | | | |
| Mannitol | | | | | 123.0 | 123.0 | | |
| Maize starch | | | | | | | 123.0 | 123.0 |
| Cro scarmello se sodium | 6.50 | 12.50 | 6.50 | 12.50 | 6.50 | 12.50 | 6.50 | 12.50 |
| Microcrystalline cellulose | 136.5 | 136.5 | 136.5 | 136.5 | 136.5 | 136.5 | 136.5 | 136.5 |
| Cro scarmello se sodium | 10.0 | 20.0 | 10.0 | 10.0 | 10.0 | 10.0 | 10.0 | 10.0 |
| Sodium stearyl fumarate | 10.0 | 10.0 | 10.0 | 10.0 | 10.0 | 10.0 | 10.0 | 10.0 |
| Tablet coating | | | | | | | | |
| Opadry pink | 18.0 | 18.0 | 18.0 | 18.0 | 18.0 | 18.0 | 18.0 | 18.0 |
| Total [mg/tablet] | 418 | 434 | 418 | 434 | 418 | 434 | 418 | 434 |

Sitagliptin HCl amorphous (with number-average particle size less than 100 microns) was prepared by mixing the sitagliptin base with 0.5 M HCl water solution and the Povidon K25. The prepared solution was sprayed onto the mixture of lactosemonohydrateor calcium hydrogen phosphate or mannitol or maize starch and a part of croscarmellose sodium. After drying and sieving, the microcrystalline cellulose and the second part of croscarmellose sodium were added to the obtained granules. After addition of sodium stearyl fumarate, mixture was compressed into tablets, which were film coated using Opadry Pink.

**Comparative Examples 9-10:**

| Example | 9 | 10 |
|---|---|---|
| Sitagliptin base | 100.0 | 100.0 |
| Microcrystalline cellulose | 124.0 | 124.0 |
| Calcium hydrogen phosphate | 123.0 | 123.0 |
| Croscarmellose sodium | 13.0 | 20.0 |
| Magnesium stearate | 8.0 | 8.0 |
| Sodium stearyl fumarate | 2.0 | 2.0 |
| Tablet coating | | |
| Opadry pink | 15.0 | 16.0 |
| Total [mg/tablet] | 385 | 392 |

The sitagliptin base was formulated into a tablet by a roller compaction process. The active ingredient, microcrystalline cellulose, calcium hydrogen phosphate, croscarmellose sodium and sodium stearylfumarate were first blended and then roller compacted (roll speed: 3-4 rpm) into ribbons. These ribbons were then milled and sieved and then the resulting granules were lubricated with the magnesium stearate and pressed into tablets. The tablets were than film coated with Opadry pink 85F34353.

**Examples 13-16:**

| Example | 13 | 14 | 15 | 16 |
|---|---|---|---|---|
| Sitagliptin base | 100.0 | 100.0 | 100.0 | 100.0 |
| 1 M HCl | 8.96* | 8.96* | 8.96* | 8.96* |
| Povidone K25 | 12.0 | 12.0 | 12.0 | 12.0 |
| Microcrystalline cellulose (Avicel PH101) | 10.0 | 20.0 | 10.0 | 10.0 |
| Butylated hydroxytoluene | 0.06 | 0.06 | 0.06 | 0.06 |
| Lactose monohydrate | 163.0 | 163.0 | | |
| Mannitol Mannidex 16700 | | | 163.0 | 163.0 |
| Croscarmellose sodium | 6.50 | 13.0 | 6.50 | 6.50 |
| Microcrystalline cellulose (Avicel PH112) | 70.98 | 70.48 | 103.48 | 70.98 |
| Mannitol | | | 100.0 | |
| Croscarmellose sodium | 10.0 | 20.0 | 14.0 | 10.0 |
| Sodium stearyl fumarate | 8.5 | 8.5 | 12.0 | 8.5 |
| Tablet coating | | | | |
| Opadry pink | 16.0 | 16.0 | 21.0 | 16.0 |
| Total [mg/tablet] | 406 | 432 | 551 | 406 |

| | | | | |
|---|---|---|---|---|
| *the amount refers to the amount of HCl without water. | | | | |

Sitagliptin HCl amorphous (with a volume mean diameter of less than 50 microns and d90 less than 200 microns, measured with Mastersizer 2000) was prepared by mixing the sitagliptin base with 1 M HCl water solution, povidone K25 and butylated hydroxytoluene (which previously dissolved in ethanol). The prepared solution was sprayed with a rotor speed of 2-3 rpm and an atomization pressure of 1,5 bar onto the mixture of lactose monohydrate or mannitol (Mannidex 16700), microcrystalline cellulose (Avicel PH101) and a part of the croscarmellose sodium. The temperature of the granulate was from 34 to 40°C. After drying and sieving, the microcrystalline cellulose (Avicel PH112) and the second part of croscarmellose sodium as well as mannitol in case of Example 15 were added to the obtained granules. After addition of sodium stearyl fumarate the mixture was compressed into tablets which were then film coated using Opadry Pink 85F34353.

## Claims

1. A pharmaceutical composition comprising amorphous sitagliptin, wherein the amorphous sitagliptin has been prepared from a solution comprising sitagliptin and a crystallization inhibitor, wherein the sitagliptin is sitagliptin hydrochloride and the crystallization inhibitor is polyvinylpyrrolidone, and wherein the composition further comprises a pharmaceutically acceptable excipient which is microcrystalline cellulose.

2. The pharmaceutical composition according to claim 1, wherein the pharmaceutical composition is prepared by a process which comprises the following steps:
a) preparing a solution of the sitagliptin and the crystallization inhibitor in a solvent; and
b) loading the obtained solution on the pharmaceutically acceptable excipient.

3. The pharmaceutical composition according to claim 1, wherein the pharmaceutical composition is prepared by a process which comprises the following steps:
a) preparing a solution of the sitagliptin and the crystallization inhibitor in a solvent;
b) spray drying the obtained solution to form a solid; and
c) mixing the obtained solid with the pharmaceutically acceptable excipient.

4. The pharmaceutical composition according to any previous claim, wherein the pharmaceutical composition is a homogeneous single layer tablet.

5. The pharmaceutical composition according to any one of claims 1 to 4, wherein the weight ratio of the sitagliptin to the crystallization inhibitor ranges from 1:0.01 to 1:1, preferably 1:0.05 to 1:0.8 and more preferably 1:0.1 to 1:0.5.

6. Pharmaceutical composition according to any one of claims 1 to 5, which comprises
(A) 10 to 35 wt.-% of sitagliptin hydrochloride,
(B) 1 to 10 wt.-%, preferably 2 to 8.0 wt.-%, of polyvinylpyrrolidone,
(C) 20 to 50 wt.-%, preferably 30 to 35 wt.-%, of microcrystalline cellulose,
(D) 10 to 40 wt.-%, preferably 20 to 30 wt.-% of a diluent selected from the group consisting of lactose, mannitol, calcium hydrogen phosphate and maize starch, preferably lactose and/or mannitol,
(E) 1 to 15 wt.-%, preferably 3.5 to 8 wt.-%, of croscarmellose sodium,
(F) 0.5 to 5 wt.-% of a lubricant, preferably magnesium stearate and/or sodium stearyl fumarate,
(G) 0 to 0.05 wt.-%, preferably 0.01 to 0.04 wt.-%, of an antioxidant, preferably butylated hydroxytoluene, and
(H) optionally further excipients.

7. Process for preparing a pharmaceutical composition according to any one of claims 1 to 6, comprising
a) providing a solution comprising sitagliptin and a crystallization inhibitor in a solvent; and
b) converting the solution provided in step a) into a particulate form.

## Patentansprüche

1. Pharmazeutische Zusammensetzung, die amorphes Sitagliptin enthält, wobei das amorphe Sitagliptin aus einer Lösung hergestellt worden ist, die Sitagliptin und einen Kristallisationsinhibitor enthält, wobei das Sitagliptin Sitagliptinhydrochlorid ist und der Kristallisationsinhibitor Polyvinylpyrrolidon ist und wobei die Zusammensetzung ferner einen pharmazeutisch annehmbaren Hilfsstoff enthält, der mikrokristalline Cellulose ist.

2. Pharmazeutische Zusammensetzung nach Anspruch 1, wobei die pharmazeutische Zusammensetzung durch ein Verfahren hergestellt wird, das die folgenden Schritte umfasst:
a) Herstellen einer Lösung von dem Sitagliptin und dem Kristallisationsinhibitor in einem Lösungsmittel und
b) Laden der erhaltenen Lösung auf den pharmazeutisch annehmbaren Hilfsstoff.

3. Pharmazeutische Zusammensetzung nach Anspruch 1, wobei die pharmazeutische Zusammensetzung durch ein Verfahren hergestellt wird, das die folgenden Schritte umfasst:
a) Herstellen einer Lösung von dem Sitagliptin und dem Kristallisationsinhibitor in einem Lösungsmittel,
b) Sprühtrocknung der erhaltenen Lösung, um einen Feststoff zu bilden und
c) Mischen des erhaltenen Feststoffes mit dem pharmazeutisch annehmbaren Hilfsstoff.

4. Pharmazeutische Zusammensetzung nach einem der vorhergehenden Ansprüche, wobei die pharmazeutische Zusammensetzung eine homogene Einschichttablette ist.

5. Pharmazeutische Zusammensetzung nach einem der Ansprüche 1 bis 4, bei der das Gewichtsverhältnis von dem Sitagliptin zu dem Kristallisationsinhibitor im Bereich von 1:0,01 bis 1:1, bevorzugt 1:0,05 bis 1:0,8 und besonders bevorzugt 1:0,1 bis 1:0,5 liegt.

6. Pharmazeutische Zusammensetzung nach einem der Ansprüche 1 bis 5, die
(A) 10 bis 35 Gew.-% Sitagliptinhydrochlorid,
(B) 1 bis 10 Gew.-%, vorzugsweise 2 bis 8,0 Gew.-%, Polyvinylpyrrolidon,
(C) 20 bis 50 Gew.-%, vorzugsweise 30 bis 35 Gew.-%, mikrokristalliner Cellulose,
(D) 10 bis 40 Gew.-%, vorzugsweise 20 bis 30 Gew.-%, eines Verdünnungsmittels ausgewählt aus der Gruppe bestehend aus Lactose, Mannit, Calciumhydrogenphosphat und Maisstärke, vorzugsweise Lactose und/oder Mannit,
(E) 1 bis 15 Gew.-%, vorzugsweise 3,5 bis 8 Gew.-%, Croscarmellose-Natrium,
(F) 0,5 bis 5 Gew.-% eines Schmiermittels, vorzugsweise Magnesiumstearat und/oder Natriumstearylfumarat,
(G) 0 bis 0,05 Gew.-%, vorzugsweise 0,01 bis 0,04 Gew.-%, eines Antioxidationsmittels, vorzugsweise Butylhydroxytoluol, und
(H) gegebenenfalls weitere Hilfsstoffe enthält.

7. Verfahren zur Herstellung einer pharmazeutischen Zusammensetzung nach einem der Ansprüche 1 bis 6, bei dem
a) eine Lösung bereitgestellt wird, die Sitagliptin und einen Kristallisationsinhibitor in einem Lösungsmittel enthält, und
b) die in Schritt a) erhaltene Lösung in eine partikuläre Form umgewandelt wird.

## Revendications

1. Composition pharmaceutique comprenant de la sitagliptine amorphe, laquelle sitagliptine amorphe a été préparée à partir d'une solution qui comprend de la sitagliptine et un inhibiteur de cristallisation, dans laquelle composition la sitagliptine se trouve à l'état de chlorhydrate et l'inhibiteur de cristallisation est de la poly(vinyl-pyrrolidone), et laquelle composition comprend en outre, en tant qu'excipient pharmacologiquement admissible, de la cellulose microcristalline.

2. Composition pharmaceutique conforme à la revendication 1, laquelle composition pharmaceutique est préparée selon un procédé qui comporte les étapes suivantes :
a) préparer une solution de sitagliptine et de l'inhibiteur de cristallisation dans un solvant,
b) et charger la solution ainsi obtenue sur l'excipient pharmacologiquement admissible.

3. Composition pharmaceutique conforme à la revendication 1, laquelle composition pharmaceutique est préparée selon un procédé qui comporte les étapes suivantes :
a) préparer une solution de sitagliptine et de l'inhibiteur de cristallisation dans un solvant,
b) faire sécher par pulvérisation la solution ainsi obtenue, pour former un solide,
c) et mélanger le solide ainsi obtenu avec l'excipient pharmacologiquement admissible.

4. Composition pharmaceutique conforme à l'une des revendications précédentes, laquelle composition pharmaceutique est un comprimé homogène, à couche simple.

5. Composition pharmaceutique conforme à l'une des revendications 1 à 4, dans laquelle la proportion pondérale de la sitagliptine à l'inhibiteur de cristallisation vaut de 1/0,01 à 1/1, de préférence de 1/0,05 à 1/0,8, et mieux encore de 1/0,1 à 1/0,5.

6. Composition pharmaceutique conforme à l'une des revendications 1 à 5, qui comprend :
A) de 10 à 35 % en poids de chlorhydrate de sitagliptine,
B) de 1 à 10 % en poids, et de préférence de 2 à 8,0 % en poids, de poly(vinyl-pyrrolidone),
C) de 20 à 50 % en poids, et de préférence de 30 à 35 % en poids, de cellulose microcristalline,
D) de 10 à 40 % en poids, et de préférence de 20 à 30 % en poids, d'un diluant choisi dans l'ensemble formé par du lactose, du mannitol, de l'hydrogénophosphate de calcium et de l'amidon de maïs, et de préférence, de lactose et/ou de mannitol,
E) de 1 à 15 % en poids, et de préférence de 3,5 à 8 % en poids, de croscarmellose sodique,
F) de 0,5 à 5 % en poids d'un lubrifiant, et de préférence, de stéarate de magnésium et/ou de fumarate de stéaryle et de sodium,
G) de 0 à 0,05 % en poids, et de préférence de 0,01 à 0,04 % en poids, d'un anti-oxydant, et de préférence, d'hydroxy-toluène butylé,
H) et en option, d'autres excipients.

7. Procédé de préparation d'une composition pharmaceutique conforme à l'une des revendications 1 à 6, comportant les étapes suivantes :
a) préparer une solution comprenant, dans un solvant, de la sitagliptine et un inhibiteur de cristallisation,
b) et convertir la solution préparée dans l'étape (a) en une forme en particules.
